# NEUE EUROPÄISCHE PATENTSCHRIFT

(11) **EP 0 838 214 B2**
(45) Veröffentlichungstag und Bekanntmachung des Hinweises auf die Entscheidung über den Einspruch: **18.06.2014**
(45) Hinweis auf die Patenterteilung: 05.01.2005
(21) Anmeldenummer: 97117560.9
(22) Anmeldetag: 10.10.1997
(51) Int. Cl.: A61K 8/49, A61Q 17/04

(54) **Kosmetische Mittel enthaltend eine Kombination von UV-Absorbern, einschliesslich Triazinderivate**
Cosmetic compositions containing a combination of UV-absorbers including a triazine derivative
Compositions cosmétiques comprenant une combinaison d'absorbants d'UV, y compris un dérivé de triazine

(30) Priorität: 22.10.1996 DE 19643515
(43) Veröffentlichungstag der Anmeldung: 29.04.1998
(73) Patentinhaber: BASF SE, 67056 Ludwigshafen (DE)
(72) Erfinder: Sperling, Karin, Dr., 67433 Neustadt (DE); Wünsch, Thomas, Dr., 67346 Speyer (DE); Westenfelder, Horst, 67435 Neustadt (DE); Trentmann, Beate, Dr., 68307 Mannheim (DE)

(56) Entgegenhaltungen:
- EP-A- 0 517 104
- EP-A- 0 570 838
- EP-A- 0 796 851
- EP-A1- 0 570 838
- EP-A1- 0 800 813
- EP-A1- 0 815 835
- EP-A1- 0 821 937
- EP-A1- 0 821 938
- EP-A1- 0 821 940
- EP-A1- 0 821 941
- A. DOMSCH: 'Die kosmetischen Präparate Band III', Bd. 4, H. ZIOLKOWSKY GMBH, AUGSBURG Seiten 620 - 469

## Beschreibung

Die vorliegende Erfindung betrifft neue kosmetische Mittel zur topischen Anwendung zum Schutz der Haut und/oder der Haare vor UV-Strahlung.

Aus EP 087 098 sind s-Triazinderivate der Formel (II) als UV-B-Filter bekannt, wobei R beispielsweise für offenkettige Alkylreste steht.

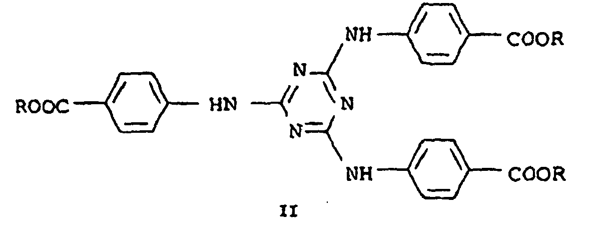

EP 517104 beschreibt ähnliche Triazinderivate der Formel (II), wobei R beispielsweise für Cycloalkylreste stehen kann und deren Verwendung als UV-Filter in kosmetischen Zubereitungen. Diese Verbindungen werden als vorteilhafter gegenüber den Verbindungen mit offenkettigen Resten R beschrieben.

EP 570 838 beschreibt neue S-Triazinderivate der Formel (III) als Lichtstabilisatoren für Kunsstoffe und als kosmetische Sonnenschutzmittel.

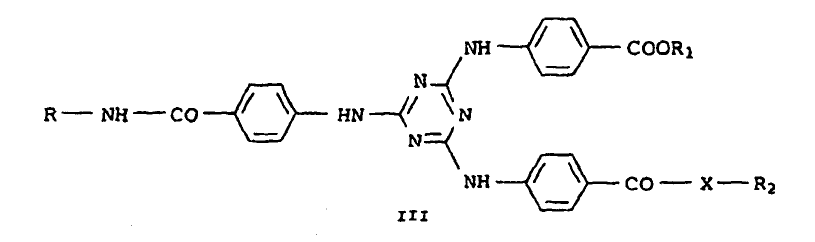

EP 685 223 beschreibt kosmetische Mittel, die einen UV-Filter der Formel (II) mit R=2-Ethylhexyl enthalten (Uvinul® T150) und außerdem als weitere Bestandteile Homomenthylsalicylat und/oder Octylsalicylat. Diese Salicylate bewirken eine schnellere Solubilisierung des Uvinul® T150 in bestimmten Lösungsmitteln

EP 685 221 beschreibt Kombinationen eines s-Triazinderivates der Formel II (R = 2-Ethylhexyl, Uvinul® T150) mit 2-Ethylhexyl-2-cyano-3,3-diphenylacrylat in Sonnenschutz-Formulierungen zur Erzielung hoher Lichtschutzfaktoren.

Obwohl die oben beschriebenen Verbindungen und Mittel bereits gute Lichtschutzeigenschaften besitzen, steigen die Anforderungen, die an kosmetische Lichtschutzmittel gestellt werden, dauernd an, so daß die Aufgabe bestand, kosmetische Lichtschutzmittel bereitzustellen, die hinsichtlich einer oder mehrerer der folgenden Eigenschaften noch bessere Werte aufweisen als die bisher bekannten Mittel:

Die wichtigsten Anforderungen an kosmetische Lichtschutzmittel sind:
1. möglichst breiter UV-Absorptionsbereich,
2. hohe spezifische Absorption in diesem Bereich,
3. Photo- und Thermostabilität,
4. Hautverträglichkeit (keine reizenden und toxischen Wirkungen auf der Haut),
5. gutes Hautgefühl und gute Haftung auf der Haut,
6. Wasserfestigkeit,
7. gute Verträglichkeit mit anderen kosmetischen Substanzen und gute Löslichkeit,
8. in kosmetischen Lösungen und Zubereitungen.

Gegenstand der Erfindung wie er für die Vertragsstaaten BE, DE, ES, FR, GB, IT, NL, AT, CH, IE und PT gültig ist, sind kosmetische Mittel zur topischen Anwendung zum Schutz der Haut und/oder der Haare vor UV-Strahlung, enthaltend

(a) einen UV-Filter A der allgemeinen Formel I, wobei 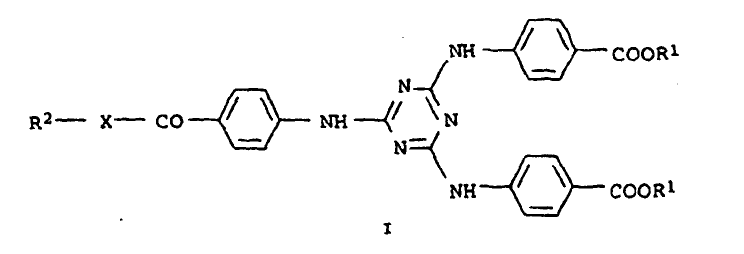 A für R¹ = 2-Ethylhexyl, R² =tert. Butyl, X = NH steht;
(b) einen weiteren UV-Filter aus der Gruppe p-Aminobenzoesäureethylester (25 Mol) ethoxyliert, p-Methoxyzimtsäure-2-ethylhexylester, 2-Phenylbenzimidazolsulfonsäure und deren Salze, 2-Hydroxy-4-methoxybenzophenon, 2-Hydroxy-4-methoxybenzophenon-5-sulfonsäure, 3-(4'-Methylbenzyliden)-d,l-campher, und 2,4,6-Trianilino- (p-carbo-2'-ethylhexyl-1'-oxy)-1,3,5-triazin,
sowie in der Kosmetik übliche Verdünnungsmittel, Hilfs- und Trägerstoffe, ausgenommen :
Öl/Wasser-Tagescreme bestehend aus 1.5 g Verbindung C, 3,5 g 2-Ethylhexyl-4-methoxycinnamat, 4,0 g Triglycerylmethylglukosedistearat, 1,0 g Glycerylstearat, 7,4 g (C₁₂-C₁₅) Alkylbenzoat, 5,0 g Avocadoöl, 5,0 g Diisopropyladipat, 0,2 g Carbomer 940, 0,3 g Imidazolidinylharnstoff, 0,2 g Methylparaben, 0,1 g Propylparaben, 0,15 g Aminomethylpropanol, 3,0 g Glycerin, destilliertes Wasser q.s. auf 100,
   oder
   Öl/Wasser-Tagescreme bestehend aus 1.5 g Verbindung C, 3,5 g 3-(4'-Methylbenzyliden)-campher 4,0 g Triglycerylmethylglukosedistearat, 1,0 g Glycerylstearat, 7,4 g (C₁₂-C₁₅)Alkylbenzoat, 5,0 g Avocadoöl, 5,0 g Diisopropyladipat, 0,2 g Carbomer 940, 0,3 g Imidazolidinylharnstoff, 0,2 g Methylparaben, 0,1 g Propylparaben, 0,15 g Aminomethylpropanol, 3,0 g Glycerin, destilliertes Wasser q.s. auf 100,
   oder
   Öl/Wasser-Tagescreme bestehend aus 1.5 g Verbindung C, 3,5 g 2-Hydroxy-4-methoxybenzophenon, 4,0 g Triglycerylmethylglukosedistearat, 1,0 g Glycerylstearat, 7,4 g (C₁₂-C₁₅)Alkylbenzoat, 5,0 g Avocadoöl, 5,0 g Diisopropyladipat, 0,2 g Carbomer 940, 0,3 g Imidazolidinylharnstoff, 0,2 g Methylparaben, 0,1 g Propylparaben, 0,15 g Aminomethylpropanol, 3,0 g Glycerin, destilliertes Wasser q.s. auf 100,
   wobei Verbindung C ein Sonnenschutzmittel der folgenden Formel ist, 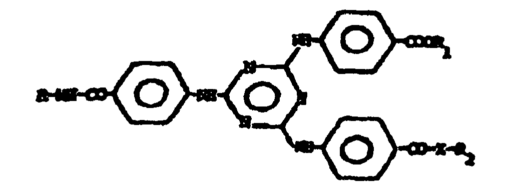 worin R = (CH₃)₃C-; R₁ = R₂ = C₄H₉-CH(C₂H₅)-CH₂-; X=O ist.

Gegenstand der Erfindung sind ebenfalls die oben genannten kosmetischen Mittel, dadurch gekennzeichnet, daß als UV-Filter (b) p-Methoxyzimtsäure-2-ethylhexylester eingesetzt wird.

Gegenstand der Erfindung in den oben genannten Vertragsstaaten ist weiterhin auch die Verwendung einer Kombination aus
(a) einem UV-Filter A der allgemeinen Formel I, wobei 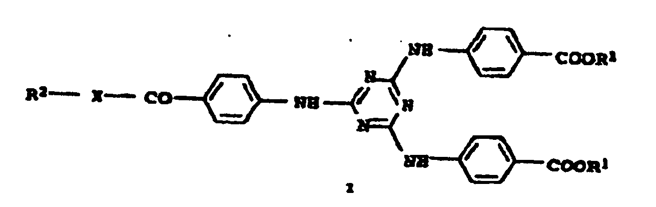 A für R¹ = 2-Ehtylhexyl, R² = tert. Butyl, X = NH steht; und
(b) einem weiteren UV-Filter aus der Gruppe p-Aminobenzoesäureethylester (25 Mol) ethoxyliert, 2-Phenylbenzimidazolsulfonsäure und deren Salze, 2-Hydroxy-4-methoxybenzophenon-5-sulfonsäure, und 2,4,6-Trianilino-(p-carbo-2'-ethylhexyl-1'-oxy)- 1,3,5-triazin als Lichtschutzmittel.

Ein weiterer Gegenstand der Erfindung, wie er für die Vertragsstaaten DK, FI, GR und SE gültig ist, sind kosmetische Mittel zur topischen Anwendung zum Schutz der Haut und/oder der Haare vor UV-Strahlung, enthalten
(a) einen UV-Filter A der aligemeinen Formel I, wobei 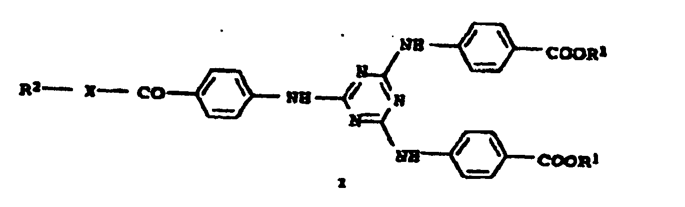 A für R¹ = 2-Ethylhexyl, R² =tert. Butyl, X = NH;
(b) einen weiteren UV-Filter aus der Gruppe p-Aminobenzoesäureethylester (25 Mol) ethoxyliert, p-Methoxyzimtsäure-2-ethylhexyslester, 2-Ethlhexyl-2-cyano-3,3-diphenylacrytat, 2-Phenylbenzimidazolsulfonsäure und deren Salze, 2-Hydroxy-4-methoxybenzophenon, 2-Hydroxy-4-methoxybenzophenon-5-sulfonsäure, 3-(4'-Methylbenzyliden)-d,I-campher, 2,4,6-Trianilino-(p-carbo-2'-ethylhexyl-1'-oxy)-1,3,5-triazin,
sowie in der Kosmetick übliche Verdünnungsmittel, Hilfs- und Trägerstoffe.

Bevorzugt sind dabei solche Mittel, die als UV-Filter (b) 2-Ethylhexyl-2-cyano-3,3-diphenylacrylat, sowie solche, die als UV-Filter (b) p-Methoxyzimtsäure-2ethylhexylester enthalten.

Gegenstand der Erfindung in den Vertragsstaaten DK, FI, GR und SE ist ferner die Verwendung einer Kombination aus
(a) einen UV-Filter A der aligemeinen Formel I, wobei 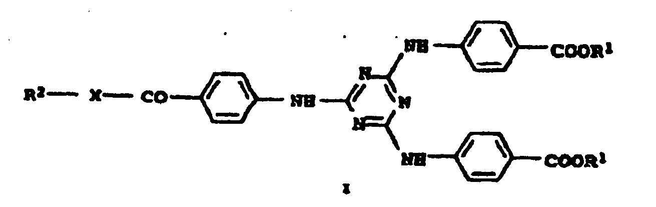 A für R¹ = 2-Ethylhexyl, R² =tert. Butyl, X = NH steht;
   und
(b) einen weiteren UV-Filter aus der Gruppe p-Aminobenzoesäureethylester (25 Mol) ethoxyliert, p-Methoxyzimtsäure-2-ethylhexyslester, 2-Phenylbenzimidazolsulfonsäure und deren Salze, 2-Hydroxy-4-methoxybenzophenon, 2-Hydroxy-4-methoxybenzophenon-5-sulfonsäure,
   3-(4'-Methylbenzyliden)-d,I-campher, 2,4,6-Trianilino-(p-carbo-2'-ethylhexyl-1'-oxy)-1,3,5-triazin,
als Lichtschutzmittel.

Die erfindungsgemäßen kosmetischen Lichtschutzmittel erweisen sich bezüglich einer oder mehrerer der oben angeführten Eigenschaften überlegen gegenüber den bisher bekannten Lichtschutzmitteln. Vor allem hinsichtlich des erreichbaren Sonnenschutzfaktors zeigen die Lichtschutzmittel überraschend gute Eigenschaften.

Kosmetische Präparate oder Zubereitungen enthalten die Verbindungen (a) und (b) im allgemeinen zu 0,1 bis.15.Gew.-%, vorzugsweise zu.5-10 Gew.%, bezogen auf die Formulierung, neben den in der Kosmetik üblichen Trägerstoffen oder Verdünnungsmitteln und gegebenenfalls üblichen kosmetischen Hilfsstoffen.

Von der Art des Trägers , Hilfsstoffes oder Verdünnungsmittel hängt es ab, ob das fertige lichtschutzmittelhaltige Präparat eine Lösung, ein Öl, eine Creme, eine Salbe, eine Lotion, ein Gel oder ein Pulver ist. Derartige Zubereitungen können beispielsweise der Zeitschrift "Seifen, Öle, Fette, Wachse", 1955, Seite 147 entnommen werden.

Üblicherweise verwendete kosmetische Hilfsstoffe, die als Zusätze in Betracht kommen, sind beispielsweise Emulgatoren, wie Fettalkoholethoxylate, Sorbitanfettsäureester oder Lanolinderivate, Verdickungsmittel, wie Carboxymethylcellulose oder vernetzte Polyacrylsäure, Konservierungsmittel und Parfüms.

Weitere Hilfsstoffe sind Stabilisatoren wie Magnesium- oder Aluminiumsalze von Fettsäuren, Komplexbildner wie EDTA, Antioxidantien wie BHT, BHA oder alpha-Tocopherol.

Kosmetisch Öle, die als Hilfsstoffe Verwendung finden sind beispielsweise Isopropylester von Fettsäuren, insbesondere Isopropylstearat, Isopropylpalmitat, Isopropylisostearat, Isopropylmyristat, Isopropyllaurat, Paraffinöl und Neutralöl.

Weitere Bestandteile der erfindungsgemäßen kosmetischen Mittel können kosmetische Wirkstoffe wie Panthenol, Bisabolol, alpha-Tocopherol, alpha-Tocopherolacetat, Aloe Vera, Algenextrakt und Hyaluronsäure sein.

Grundlage für Sonnenschutzöle sind beispielsweise pflanzliche Öle, wie Erdnußöl, Olivenöl, Sesamöl, Baumwollsamenöl, Kokosöl, Traubenkernöl, Ricinusöl oder Mineralöle, wie Vaselineöl, oder insbesondere flüssiges Paraffin, synthetische Fettsäureester und Glyceride. Grundlage für Salben sind beispielsweise Vaseline, Lanolin Eucerin oder Polyethylenglykole.

Grundlagen für Cremes sind beispielsweise fettreiche Cremes, Glycerin-, Polysaccharid-, Tylosecreme, für Cremes auf Basis von Fetten und Wachsen Cetylalkohol, Lanolincreme, Kakaobutter, Bienenwachs, Stearinsäure, Stearylalkohol, Glycerinmonostearat, native oder mineralische Öle und Fette.

Grundlagen für Emulsionen sind beispielsweise Mischungen aus Stearylglykol, einem pflanzlichen und/oder Mineralöl, wie Mandelöl, Paraffinöl und Vaseline, und Wasser oder Mischungen aus Ethylalkohol, Wasser, Lanolin und Tragant, oder Mischungen aus Ethylalkohol, Stearin, Wasser, Tragant und Glycerin oder Mischungen aus Stearinsäure, Paraffinöl, Propyl- oder Isopropylalkohol und Wasser.

Die Erfindung ist in den folgenden Beispielen weiter veranschaulicht. "UV Filter A" ist die Verbindung der Formel (I), worin R¹=2-Ethylhexyl, R² = tert.-Butyl und X=NH.

Alle Zahlenangaben in den folgenden Rezepturen sind in Gramm.

### Beispiel 1

### Herstellung eines kosmetischen Lichtschutzmittels in Form eines fettfreien Gels

| Produkt: | Sonnenschutz-Gel-fettfrei |
|---|---|
| 0,40 | Acrylates/C10-C30 Alkyl Acrylate Cross |
| 0,25 | Hydroxyethyl Cellulose |
| 8,00 | Octyl Methoxycinnamate |
| 1,00 | 4-Methylbenzylidene Camphor |

| Produkt: | Sonnenschutz-Gel-fettfrei |
|---|---|
| 2,00 | UV-Filter A |
| 0,20 | Disodium EDTA |
| q.s | Water |
| 5,00 | Glycerin |
| 0,15 | Fragrance |
| 0,30 | Imidazolidinyl Urea |
| 0,25 | Sodium Methylparaben |
| 0,15 | Sodium Propylparaben |
| 5,00 | PEG-25 PABA |
| 0,10 | Sodium Hydroxide |

Das Gel besitzt einen Sonnenschutzfaktor (SPF, gemessen mit Optometrics SPF 290) von 13.

### Vergleichsversuch:

Eine ansonsten gleiche Formulierung, bei der anstatt UV-Filter A die gleiche Menge Uvinul T150 verwendet wurde, besitzt einen SPF von 8.

### Beispiel 2

### Herstellung eines kosmetischen Lichtschutzmittels in Form einer Sonnencreme

| Produkt: | Sonnencreme |
|---|---|
| 6,00 | PEG-7-Hydrogenated Castor Oil |
| 5,00 | Zinc Oxide |
| 5,00 | Isopropyl Palmitate |
| 6,00 | Mineral Oil |
| 3,00 | Octylsalicylat |
| 0,50 | Tocopheryl Acetate |
| 8,00 | Octyl Methoxycinnamate |
| 1,00 | 4-Methylbenzylidene Camphor |
| 2,00 | UV-Filter A |
| 0,60 | Magnesium Stearate |
| 2,00 | PEG-45/Dodecyl Glycol Copolymer |
| 0,25 | Methylpyraben |
| 0,15 | Propylparaben |
| 5,00 | Imidazolidinyl Urea |
| 0,15 | Fragrance |

| Produkt: | Sonnencreme |
|---|---|
| 0,20 | Disodium EDTA |
| q.s | Aqua |

Die Creme besitzt einen Sonnenschutzfaktor (SPF, gemessen mit Optometrics SPF 290) von 16.

### Vergleichsversuch:

Eine ansonsten gleiche Formulierung, bei der anstatt UV-Filter A die gleiche Menge Uvinul T150 verwendet wurde, besitzt einen SPF von 12.

### Beispiel 3 (nur für die Vertragsstaaten DK, FI, GR und SE)

### Herstellung eines kosmetischen Lichtschutzmittels in Form einer Sonnenmilch

| Produkt: | LSF 6 Sonnenmilch |
|---|---|
| 6,00 | PEG-7-Hydrogenated Castor Oil |
| 5,00 | Isopropyl Palmitate |
| 10,00 | Mineral Oil |
| 3,00 | Caprylic/Capric Triglyceride |
| 0,60 | Magnesium Stearate |
| 3,00 | Octocrylene |
| 0,50 | Tocopheryl Acetate |
| 2,00 | PEG-45/Dodecyl Glycol Copolymer |
| 0,05 | Tocopherol |
| 2,00 | UV-Filter A |
| q.s | Aqua |
| 0,30 | Glycerin |
| 0,70 | Magnesium Sulfate |
| 0,25 | Methylparaben |
| 0,15 | Propylparaben |
| 0,15 | Fragrance |

Das Gel besitzt einen Sonnenschutzfaktor (SPF, gemessen mit Optometrics SPF 290) von 8.

### Vergleichsversuch:

Eine ansonsten gleiche Formulierung, bei der anstatt UV-Filter A die gleiche Menge Uvinul T150 verwendet wurde, besitzt einen SPF von 4.

### Beispiel 4

### Herstellung eines kosmetischen Lichtschutzmittels in Form eines Lippensunblock

| Produkt: | Lippensunblock |
|---|---|
| 48,00 | Eucerinum anhydrcum |
| 2,00 | Bees Wax |
| 2,00 | UV-Filter A |
| 4,00 | Pentaerythrithyl Stearate/Caprate/Caprylate |
| 2,00 | Microcrystalline Wax |
| 10,00 | Octyl Methoxycinnamate |
| 3,00 | Glyceryl Stearate SE |
| 10,00 | Glycerin |
| 2,00 | Quaternium-18 Bentonite |
| 1,50 | PEG-45/Dodecyl Glycol Copolymer |
| 5,00 | Zinc Oxide |
| 10,00 | Titanium Dioxide |

Der Lippensunblock besitzt einen Sonnenschutzfaktor (SPF, gemessen mit Optometrics SPF 290) von 35.

### Vergleichsversuch:

Eine ansonsten gleiche Formulierung, bei der anstatt UV-Filter A die gleiche Menge Uvinul T150 verwendet wurde, besitzt einen SPF von 28.

## Patentansprüche (Patentansprüche für folgende(n) Vertragsstaat(en): BE, DE, ES, FR, GB, IT, NL, AT, CH, IE, PT)

1. Kosmetische Mittel zur topischen Anwendung zum Schutz der Haut und/oder der Haare vor UV-Strahlung, enthaltend
(a) einen UV-Filter A der allgemeinen Formel I, wobei 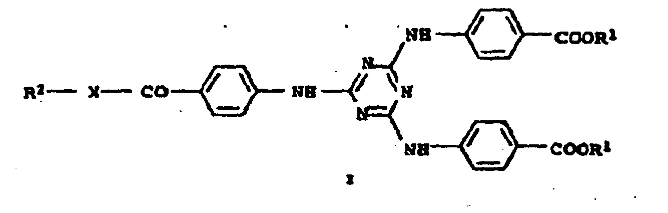 A für R¹ = 2-Ethylhexyl, R² =tert. Butyl, X = NH;
(b) einen weiteren UV-Filter aus der Gruppe p-Aminobenzoesäureethylester (25 Mol) ethoxyliert, p-Methoxyzimtsäure-2-ethylhexylester, 2-Ethylhexyl-2-cyano-3,3-diphenylacrylat, 2-Phenylbenzimidazolsulfonsäure und deren Salze, 2-Hydroxy-4-methoxybenzophenon, 2-Hydroxy-4-methoxybenzophenon-5-sulfonsäure, 3-(4'-Methylbenzyliden)-d,l-campher, 2,4,6-Trianilino-(p-carbo-2'-ethylhexyl-1'-oxy)-1,3,5-triazin,
sowie in der Kosmetik übliche Verdünnungsmittel, Hilfs- und Trägerstoffe,
ausgenommen:
Öl/Wasser-Tagescreme bestehend aus 1.5 g Verbindung C, 3,5 g 2-Ethylhexyl-4-methoxycinnamat, 4,0 g Triglycerylmethylglukosedistearat, 1,0 g Glycerylstearat, 7,4 g (C₁₂-C₁₅) Alkylbenzoat, 5,0 g Avocadoöl, 5,0 g Diisopropyladipat, 0,2 g Carbomer 940, 0,3 g Imidazolidinylharnstoff, 0,2 g Methylparaben, 0,1 g Propylparaben, 0,15 g Aminomethylpropanol, 3,0 g Glycerin, destilliertes Wasser q.s. auf 100,
oder
Öl/Wasser-Tagescreme bestehend aus 1.5 g Verbindung C, 3,5 g 3-(4'-Methylbenzyliden)-campher 4,0 g Triglycerylmethylglukosedistearat, 1,0 g Glycerylstearat, 7,4 g (C₁₂-C₁₅)Alkylbenzoat, 5,0 g Avocadoöl, 5,0 g Diisopropyladipat, 0,2 g Carbomer 940, 0,3 g Imidazolidinylharnstoff, 0,2 g Methylparaben, 0,1 g Propylparaben, 0,15 g Aminomethylpropanol, 3,0 g Glycerin, destilliertes Wasser q.s. auf 100,
oder
Öl/Wasser-Tagescreme bestehend aus 1.5 g Verbindung C, 3,5 g 2-Hydroxy-4-methoxybenzophenon, 4,0 g Triglycerylmethylglukosedistearat, 1,0 g Glycerylstearat, 7,4 g (C₁₂-C₁₅)Alkylbenzoat, 5,0 g Avocadoöl, 5,0 g Diisopropyladipat, 0,2 g Carbomer 940, 0,3 g Imidazolidinylharnstoff, 0,2 g Methylparaben, 0,1 g Propylparaben, 0,15 g Aminomethylpropanol, 3,0 g Glycerin, destilliertes Wasser q.s. auf 100,
wobei Verbindung C ein Sonnenschutzmittel der folgenden Formel ist, 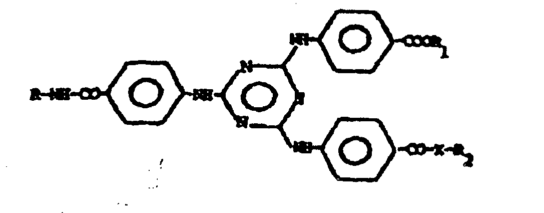 worin R = (CH₃)₃C-; R₁ = R₂ = C₄H₉-CH(C₂H₅)-CH₂-; X=O ist.

2. Mittel nach Anspruch 1, **dadurch gekennzeichnet, daß** als UV-Filter (b) , p-Methoxyzimtsäure-2-Ethylhexylester eingesetzt wird.

3. Verwendung einer Kombination aus
(a) einen UV-Filter A der allgemeinen Formel I, wobei 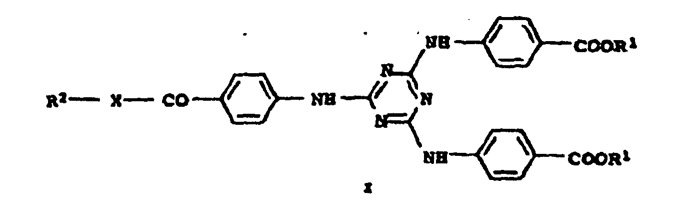 A für R¹ = 2-Ethylhexyl, R² =tert. Butyl, X = NH steht;
und
(b) einen weiteren UV-Filter aus der Gruppe p-Aminobenzoesäureethylester (25 Mol) ethoxyliert, 2-Phenylbenzimidazolsulfonsäure und deren Salze, 2-Hydroxy-4-methoxybenzophenon-5-sulfonsäure, 2,4,6-trianilino-(p-carbo-2'-ethylhexyl-1'-oxy)-1,3,5-triazin,
als Lichtschutzmittel.

## Patentansprüche (Patentansprüche für folgende(n) Vertragsstaat(en): DK, FI, GR, SE)

1. Kosmetische Mittel zur topischen Anwendung zum Schutz der Haut und/oder der Haare vor UV-Strahlung, enthaltend
(a) einen UV-Filter A der allgemeinen Formel I, wobei 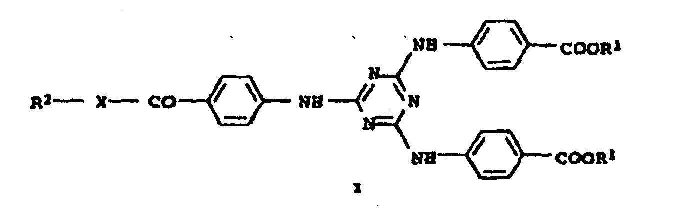 A für R¹ = 2-Ethylhexyl, R² =tert. Butyl, X = NH;
(b) einen weiteren UV-Filter aus der Gruppe p-Aminobenzoesäureethylester (25 Mol) ethoxyliert, p-Methoxyzimtsäure-2-ethylhexylester, 2-Ethylhexyl-2-cyano-3,3-diphenylacrylat, 2-Phenylbenzimidazolsulfonsäure und deren Salze, 2-Hydroxy-4-methoxybenzophenon, 2-Hydroxy-4-methoxybenzophenon-5-sulfonsäure, 3-(4'-Methylbenzyliden)-d,l-campher, 2,4,6-Trianilino-(p-carbo-2'-ethylhexyl-1'-oxy)-1,3,5-triazin,
sowie in der Kosmetik übliche Verdünnungsmittel, Hilfs- und Trägerstoffe,

2. Mittel nach Anspruch 1, **dadurch gekennzeichnet, daß** als UV-Filter (b) 2-Ethylhexyl-2-cyano-3,3-diphenylacrylat eingesetzt wird.

3. Mittel nach Anspruch 1, **dadurch gekennzeichnet, daß** als UV-Filter (b) p-Methoxyzimtsäure-2-Ethylhexylester eingesetzt wird.

4. Verwendung einer Kombination aus
(a) einen UV-Filter A der allgemeinen Formel I, wobei 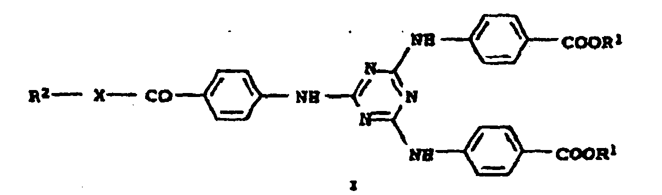 A für R¹ = 2-Ethylhexyl, R² =tert. Butyl, X = NH steht;
und
(b) einem weiteren UV-Filter aus der Gruppe p-Aminobenzoesäureethylester (25 Mol) ethoxyliert, p-Methoxyzimtsäure-2-ethylhexylester, 2-Phenylbenzimidazolsulfonsäure und deren Salze, 2-Hydroxy-4-methoxybenzophenon, 2-Hydroxy-4-methoxybenzophenon-5-sulfonsäure,
3-(4'-Methylbenzyliden)-d,l-campher, 2,4,6-Trianilino-(p-carbo-2'-ethylhexyl-1'-oxy)-1,3,5-triazin,
als Lichtschutzmittel.

## Claims (Claims for the following Contracting State(s): BE, DE, ES, FR, GB, IT, NL, AT, CH, IE, PT)

1. A cosmetic composition for topical use for protecting the skin and/or hair from UV radiation, comprising
(a) a UV filter A of the formula I, where 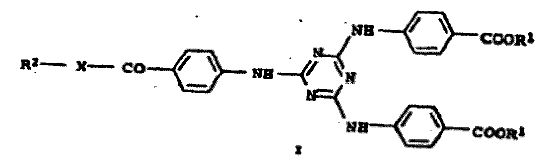 A for R¹ = 2-ethylhexyl, R² - tert-butyl, X = NH;
(b) another UV filter from the group of ethyl p-aminobenzoate (25 mol) ethoxylated, 2-ethylhexyl p-methoxycinnamate, 2-phenylbenzimidazolesulfonic acid and its salts, 2-hydroxy-4-methoxybenzophenone, 2-hydroxy-4-methoxybenzophenone-5-sulfonic acid, 3-(4'-methylbenzylidene)-d,1-camphor, 2,4,6-trianilino(p-carbo-2'-ethylhexyl-1'-oxy)-1,3,5-triazine,
as well as diluents, auxiliaries and carriers customary in cosmetics,
except for:
oil/water day cream consisting of 1.5 g of compound C, 3.5 g of 2-ethylhexyl 4-methoxycinnamate, 4.0 g of triglyceryl methylglucose distearate, 1.0 g of glyceryl stearate, 7.4 g of (C₁₂-C₁₅)-alkyl benzoate, 5.0 g of advocado oil, 5.0 g of diisopropyl adipate, 0.2 g of carbomer 940, 0.3 g of imidazolidinylurea, 0.2 g of methylparaben, 0.1 g of propylparaben, 0.15 g of aminomethylpropanol, 3.0 g of glycerol, distilled water q.s. ad 100,
or
oil/water day cream consisting of 1.5 g of compound C, 3.5 g of 3-(4'-methylbenzylidene)camphor, 4.0 g of triglyceryl methylglucose distearate, 1.0 g of glyceryl stearate, 7.4 g of (C₁₂-C₁₅)-alkyl benzoate, 5.0 g of avocado oil, 5.0 g of diisopropyl adipate, 0.2 g of carbomer 940, 0.3 g of imidazolidinylurea, 0.2 g of methylparaben, 0.1 g of propylparaben, 0.15 g of aminomethylpropanol, 3.0 g of glycerol, distilled water q.s. ad 100,
or
oil/water day cream consisting of 1.5 g of compound C, 3.5 g of 2-hydroxy-4-methoxybenzophenone, 4.0 g of triglyceryl methylglucose distearate, 1.0 g of glyceryl stearate, 7.4 g of (C₁₂-C₁₅)-alkyl benzoate, 5.0 g of avocado oil, 5.0 g of diisopropyl adipate, 0.2 g of carbomer 940, 0.3 g of imidazolidinylurea, 0.2 g of methylparaben, 0.1 g of propylparaben, 0.15 g of aminomethylpropanol, 3.0 g of glycerol, distilled water q.s. ad 100,
where compound C is a sun protection agent of the formula below 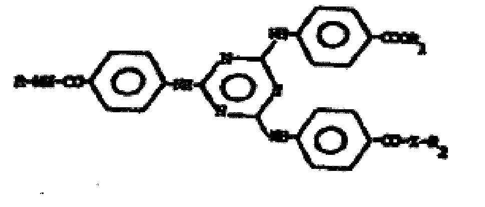
in which R = (CH₃)₃C-; R₁ = R₂ = C₄H₉-CH(C₂H₅)-CH₂-; X = O.

2. A composition according to claim 1, wherein 2-ethylhexyl p-methoxycinnamate is employed as UV filter (b).

3. The use of a combination of
(a) a UV filter A of the formula I, where 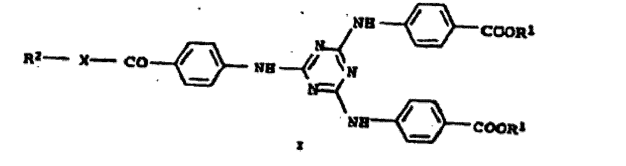 A is R¹ = 2-ethylhexyl, R² - tert-butyl, X = NH;
and
(b) another UV filter from the group of ethyl p-aminobenzoate (25 mol) ethoxylated, 2-phenylbenzimidazolesulfonic acid and its salts, 2-hydroxy-4-methoxybenzophenone-5-sulfonic acid, 2,4,6-trianilino(p-carbo-2'-ethylhexyl-1'-oxy)-1,3,5-triazine,
as sunscreen.

## Claims (Claims for the following Contracting State(s): DK, FI, GR, SE)

1. A cosmetic composition for topical use for protecting the skin and/or hair from UV radiation, comprising
(a) a UV filter A of the formula I, where 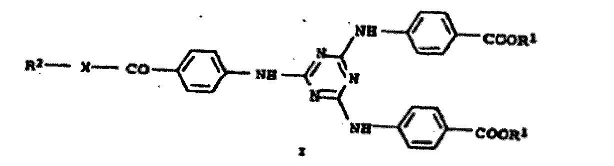 A for R¹ = 2-ethylhexyl, R² - tert-butyl, X = NH;
(b) another UV filter from the group of ethyl p-aminobenzoate (25 mol) ethoxylated, 2-ethylhexyl p-methoxycinnamate, 2-ethylhexyl 2-cyano-3,3-diphenylacrylate, 2-phenylbenzimidazolesulfonic acid and its salts, 2-hydroxy-4-methoxybenzophenone, 2-hydroxy-4-methoxybenzophenone-5-sulfonic acid, 3-(4'-methylbenzylidene)-d,1-camphor, 2,4,6-trianilino(p-carbo-2'-ethylhexyl-1'-oxy)-1,3,5-triazine,
as well as diluents, auxiliaries and carriers customary in cosmetics.

2. A composition as claimed in claim 1, wherein 2-ethylhexyl 2-cyano-3,3-diphenylacrylate is employed as UV filter (b).

3. A composition as claimed in claim 1, wherein 2-ethylhexyl p-methoxycinnamate is employed as UV filter (b).

4. The use of a combination of
(a) a UV filter A of the formula I, where 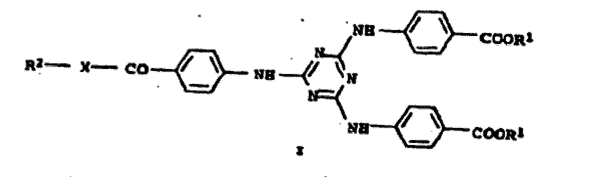 A is R¹ = 2-ethylhexyl, R² = tert-butyl, X = NH;
and
(b) another UV filter from the group of ethyl p-aminobenzoate (25 mol) ethoxylated, 2-ethylhexyl p-methoxycinnamate, 2-phenylbenzimidazolesulfonic acid and its salts, 2-hydroxy-4-methoxybenzophenone, 2-hydroxy-4-methoxybenzophenone-5-sulfonic acid, 3-(4'-methylbenzylidene)-d,1-camphor, 2,4,6-trianilino(p-carbo-2'-ethylhexyl-1'-oxy)-1,3,5-triazine,
as sunscreen.

## Revendications (Revendications pour l'(les) Etat(s) contractant(s) suivant(s): BE, DE, ES, FR, GB, IT, NL, AT, CH, IE, PT)

1. Composition cosmétique destinée à l'application topique pour la protection de la peau et/ou des cheveux contre le rayonnement UV, contenant
(a) un filtre UV A de formule générale I, 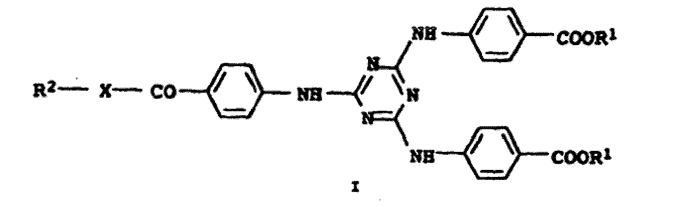 dans A R¹ représentant le groupe 2-éthylhexyle, R² représentant le groupe tert-butyle, X représentant NH ;
(b) un autre filtre UV choisi dans le groupe constitué par le p-aminobenzoate d'éthyle éthoxylé (25 moles), le p-méthoxycinnamate de 2-éthylhexyle, l'acide 2-phénylbenzimidazolesulfonique et ses sels, la 2-hydroxy-4-méthoxybenzophénone, l'acide 2-hydroxy-4-méthoxybenzophénone-5-sulfonique, le 3-(4'-méthylbenzylidène)-d,1-camphre, la 2,4,6-trianilino-(p-carbo-2'-éthylhexyl-1'-oxy)-1,3,5-triazine,
ainsi que des diluants, adjuvants et véhicules usuels en cosmétique,
à l'exclusion :
d'une crème de jour huile/eau constituée de 1,5 g de composé C, 3,5 g de 4-méthoxycinnamate de 2-éthylhexyle, 4,0 g de distéarate de triglycérylméthylglucose, 1,0 g de stéarate de glycéryle, 7,4 g de benzoate d'alkyle(C₁₂-C₁₅), 5,0 g d'huile d'avocat, 5,0 g d'adipate de diisopropyle, 0,2 g de carbomère 940, 0,3 g d'imidazolidinylurée, 0,2 g de méthylparabène, 0,1 g de propylparabène, 0,15 g d'aminométhyl-propanol, 3,0 g de glycérol, eau distillée q.s. pour 100,
ou
d'une crème de jour huile/eau constituée de 1,5 g de composé C, 3,5 g de 3-(4'-méthylbenzylidène)-camphre, 4,0 g de distéarate de triglycérylméthyl-glucose, 1,0 g de stéarate de glycéryle, 7,4 g de benzoate d'alkyle (C₁₂-C₁₅), 5,0 g d'huile d'avocat, 5,0 g d'adipate de diisopropyle, 0,2 g de carbomère 940, 0,3 g d'imidazolidinylurée, 0,2 g de méthylparabène, 0,1 g de propylparabène, 0,15 g d'aminométhylpropanol, 3,0 g de glycérol, eau distillée q.s. pour 100,
ou
d'une crème de jour huile/eau constituée de 1,5 g de composé C, 3,5 g de 2-hydroxy-4-méthoxy-benzophénone, 4,0 g de distéarate de triglycérylméthylglucose, 1,0 g de stéarate de glycéryle, 7,4 g de benzoate d'alkyle(C₁₂-C₁₅). 5,0 g d'huile d'avocat, 5,0 g d'adipate de diisopropyle, 0,2 g de carbomère 940, 0,3 g d'imidazolidinylurée, 0,2 g de méthylparabène, 0,1 g de propylparabène, 0,15 g d'aminométhyl-propanol, 3,0 g de glycérol, eau distillée q.s. pour 100,
le composé C étant un agent de protection solaire de formule suivante 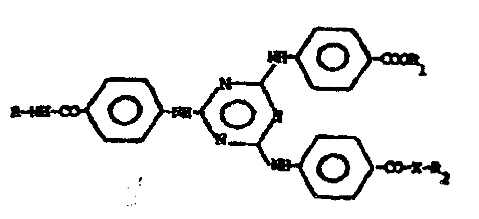 dans laquelle R est (CH₃)₃C- ;
R₁ = R₂ = C₄Hg-CH(C₂H₅)-CH₂- ; X est = O.

2. Composition selon la revendication 1, **caractérisée en ce qu'**on utilise comme filtre UV (b) le p-méthoxycinnamate de 2-éthylhexyle.

3. Utilisation d'une association de
a) un filtre UV A de formule générale I, 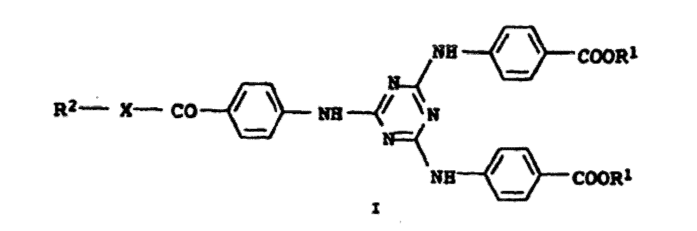
dans A R¹ représentant le groupe 2-éthylhexyle, R² représentant le groupe tert-butyle, X représentant NH ;
et
(b) un autre filtre UV choisi dans le groupe constitué par le p-aminobenzoate d'éthyle éthoxylé (25 moles), l'acide 2-phénylbenzimidazolesulfonique et ses sels, l'acide 2-hydroxy-4-méthoxybenzophénone-5-sulfonique, la 2,4,6-trianilino-(p-carbo-2'-éthylhexyl-1'-oxy)-1,3,5-triazine,
en tant que photoprotecteur.

## Revendications (Revendications pour l'(les) Etat(s) contractant(s) suivant(s): DK, FI, GR, SE)

1. Composition cosmétique destinée à l'application topique pour la protection de la peau et/ou des cheveux contre le rayonnement UV, contenant
(a) un filtre UV A de formule générale I, 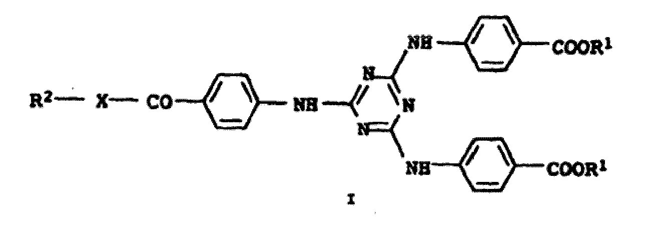 dans A R¹ représentant le groupe 2-éthylhexyle, R² représentant le groupe tert-butyle, X représentant NH ;
(b) un autre filtre UV choisi dans le groupe constitué par le p-aminobenzoate d'éthyle éthoxylé (25 moles), le p-méthoxycinnamate de 2-éthylhexyle, le 2-cyano-3,3-diphénylacrylate de 2-éthylhexyle, l'acide 2-phénylbenzimidazolesulfonique et ses sels, la 2-hydroxy-4-méthoxy-benzophénone, l'acide 2-hydroxy-4-méthoxy-benzophénone-5-sulfonique, le 3-(4'-méthyl-benzylidène)-d,1-camphre, la 2,4,6-trianilino-(p-carbo-2'-éthylhexyl-1'-oxy)-1,3,5-triazine,
ainsi que des diluants, adjuvants et véhicules usuels en cosmétique.

2. Composition selon la revendication 1, **caractérisée en ce qu'**on utilise comme filtre UV (b) le 2-cyano-3,3-diphénylacrylate de 2-éthylhexyle.

3. Composition selon la revendication 1, **caractérisée en ce qu'**on utilise comme filtre UV (b) le p-méthoxycinnamate de 2-éthylhexyle.

4. Utilisation d'une association de
a) un filtre UV A de formule générale I, 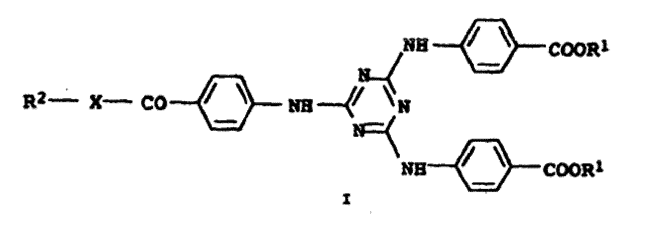 dans A R¹ représentant le groupe 2-éthylhexyle, R² représentant le groupe tert-butyle, X représentant NH ;
et
(b) un autre filtre UV choisi dans le groupe constitué par le p-aminobenzoate d'éthyle éthoxylé (25 moles), le p-méthoxycinnamate de 2-éthylhexyle, l'acide 2-phénylbenzimidazolesulfonique et ses sels, la 2-hydroxy-4-méthoxybenzophénone, l'acide 2-hydroxy-4-méthoxybenzophénone-5-sulfonique,
le 3-(4'-méthylbenzylidène)-d,1-camphre, la 2,4,6-trianilino-(p-carbo-2'-éthylhexyl-1'-oxy)-1,3,5-triazine,
en tant que photoprotecteur.
